# EUROPEAN PATENT SPECIFICATION

(11) **EP 1 169 468 B1**
(45) Date of publication and mention of the grant of the patent: **14.03.2012**
(21) Application number: 99915272.1
(22) Date of filing: 05.04.1999
(51) Int. Cl.: C12P 17/10, C07D 205/08

(54) **STEREOSELECTIVE MICROBIAL REDUCTION FOR THE PREPARATION OF 1-(4-FLUOROPHENYL)-3(R)-¬3(S)-HYDROXY-3-(4-FLUOROPHENYL)PROPYL) -4(S)-(4-HYDROXYPHENYL)-2-AZETIDINONE**
STEREOSELEKTIVE MIKROBIELLE REDUKTION FÜR DIE HERSTELLUNG VON 1-(4-FLUOROPHENYL)-3(R)-¬3(S)-HYDROXY-3-(4-FLUOROPHENYL)PROPYL) -4(S)-(4-HYDROXYPHENYL)-2-AZETIDINON
REDUCTION MICROBIENNE STEREOSELECTIVE PERMETTANT DE PREPARER 1-(4-FLUOROPHENYL)-3(R)- 3(S)-HYDROXY-3-(4-FLUOROPHENYL)PROPYL) -4(S)-(4-HYDROXYPHENYL)-2-AZETIDINONE

(43) Date of publication of application: 09.01.2002
(60) Divisional application: 10012143.3
(73) Proprietor: Schering Corporation, Kenilworth, NJ 07033-0530 (US)
(72) Inventor: HOMANN, Michael, J., Clinton, NJ 08809 (US); PREVITE, Edward, North Brunswick, NJ 08902 (US)
(74) Representative: Hussain, Deeba
(86) International application number: PCT/US1999/007445
(87) International publication number: WO 2000/060107

(56) References cited:
- WO-A-97/16424
- DATABASE WPI Section Ch, Week 198632 Derwent Publications Ltd., London, GB; Class B03, AN 1986-208964 XP002123973 & JP 61 141894 A (SANKYO CO LTD), 28 June 1986 (1986-06-28)
- SANTANIELLO E. ET AL.: "The Biocatalytic Approach to the Preparation of Enantiomerically Pure Chiral Building Blocks." CHEM. REV., vol. 92, 1992, pages 1071-1087, XP002123971
- BELAN A. ET AL.: "Use of Biological Systems for the Preparation of Chiral Molecules." J. ORG. CHEM., vol. 52, 1987, pages 256-260, XP002123972

## Description

### BACKGROUND OF THE INVENTION

1-(4-Fluorophenyl)-3(R)-[3(S)-hydroxy-3-(4-fluorophenyl)-propyl)]-4(S)-(4-hydroxyphenyl)-2-azetidinone is disclosed as a cholesterol lowering agent in WO 95/08532, published March 30, 1995. U.S. Patent 5,618,707 discloses stereoselective microbial reduction of a keto intermediate (4-(4-fluoro-benzoyl)butyric acid or a phenyloxazolidinone conjugate thereof) used in the preparation of the azetidinone to the corresponding hydroxy intermediate using the microorganism *Zygosaccharomyces bailii* or *Schizosaccharomyces octosporus.*

WO97116424 describes the chemical reduction of 1- (4-fluorophenyl)- 3 (R)- [3-oxo-3- (4-fluorophenyl) propyl)]-4 (S)- (4-hydroxyphenyl)-2- azetidinone to 1- (4-fluorophenyl)-3 (R)-[3(S)-hydroxy-3- (4-fluorophenyl)propyl)]-4 (S)- (4-hydroxyphenyl)-2-azetidinone and JP61141894 describes the reduction of keto compounds by microorganisms for the production of optically active beta lactam compounds.

### SUMMARY OF THE INVENTION

The present invention relates to a process for the microbiological reduction of carbonyl groups which comprises the use of microorganisms (obtained from environmental sources and culture collections, e.g., the American Type Culture Collection (ATCC)) in medium, medium and buffer, medium and solvent, or medium and a mixture of buffer and solvent to which a ketone compound can be added so that a compound having a hydroxy group of desired stereochemistry can be formed, accumulated and isolated.

In particular, the present invention relates to a process for the stereoselective reduction of 1-(4-fluorophenyl)-3(R)-[3-oxo-3-(4-fluorophenyl)propyl)]-4(S)-(4-hydroxyphenyl)-2-azetidinone to 1-(4-fluoro-phenyl)-3(R) -[3(S)-hydroxy-3-(4-fluorophenyl)-propyl)]-4(S)-(4-hydroxyphenyl)-2-azetidinone comprising adding 1-(4-fluoro-phenyl)-3(R)-[3-oxo-3-(4-fluorophenyl)-propyl)]-4(S)-(4-hydroxyphenyl)-2-azetidinone to a micoorganism in medium, medium and buffer, medium and solvent, or medium and a mixture of buffer and solvent, incubating the resulting mixture, and isolating 1-(4-fluoro-phenyl)-3(R) -[3(S)-hydroxy-3-(4-fluorophenyl)-propyl)]-4(S)-(4-hydroxyphenyl)-2-azetidinone.

Microorganisms selected from the group consisting of the following genera have been found to be useful in the reduction of this invention: *Aspergillus, Curvularia, Doratomyces, Geotrichum, Mortierella, Mucor, Saccharomyces, Scytalidium, Pichia, Torulaspora, Neurospora* and *Rhodococcus.* The following species of the above genera are preferred: *Aspergillus niveus, Curvularia lunata, Doratomyces stemonitis, Geotrichum candidum, Mortierella isabellina, Mucor racemosus* and *circinelloides, Saccharomyces cerevisiae* and *uvarum, Scytalidium lignicola, Pichia methanolitica, Torulaspora fermentati* and *species, Neurospora crassa* and *Rhodococcus erythropolis, fascians, rhodochrous* and *species.*

In particular, the present invention relates to a process for the microbiological reduction of the carbonyl group of 1-(4-fluorophenyl)-3(R)-[3-oxo-3-(4-fluorophenyl)propyl)]-4(S)-(4-hydroxyphenyl)-2-azetidinone (Formula II, below) comprising adding said compound to a microorganism in medium, medium and buffer, medium and solvent, or medium and a mixture of buffer and solvent, especially wherein the microorganism is *Rhodococcus fascians* ATCC No. 202210 or fungal isolate *Geotrichum candidum* ATCC No. 74487, incubating the resulting mixture, and isolating 1-(4-fluorophenyl)-3(R)-[3(S)-hydroxy-3-(4-fluorophenyl)-propyl)]-4(S)-(4-hydroxyphenyl)-2-azetidinone (Formula I, below).

Viable cultures of the microorganism and the fungal isolate have been deposited in the collection of the American Type Culture Collection, 10801 University Boulevard, Manassas, VA 20110-2209, where the microorganism has been assigned accession number ATCC 202210 and fungal isolate has been assigned accession number ATCC 74487. Should a depositied culture become lost, destroyed or non-viable during the longer of the thirty (30) year period from the date the culture was deposited or the five (5) year period after the last request for the deposited culture or the effective life of the patent which issues from this application, the culture will be replaced, upon notice, by applicants or assignee(s) of this application. Subcultures of *Rhodococcus fascians* ATCC No. 202210 and *Geotrichum candidum* ATCC 74487 are available during the pendency of this application to one determined by the Commissioner of Patents and Trademarks to be entitled thereto under 37 C.F.R. 1.14 and 35 U.S.C. 122 and will be available to the public without restriction once a patent based on this application is granted. Use of the microoganism and fungal isolate is dependent on the US Patent Laws.

### DETAILED DESCRIPTION

This invention relates to a method for performing the following stereospecific reduction using a microorganism.

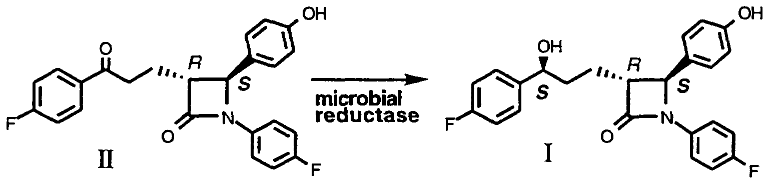

The microbiological reduction is carried out by adding the ketone substrate of formula II, above, to medium, medium and buffer, medium and solvent, or medium and a mixture of buffer and solvent containing microorganisms. The incubation may be conducted at temperatures in the range from between about 20°C and about 40°C, preferably 30°C, while adjusting the initial pH value of the reaction in the range from between about 5.0 and about 9.0, preferably 7.0.

The initial concentration of compound II in the reaction may vary from between about 0.5 g/l and about 10.0 g/l, and is preferably 2-4.0 g/l.

Suitable fermentation media, buffers and solvents are known to those skilled in the art. Fermentation media typically contain a carbon and nitrogen source or mixtures thereof, using such ingredients as yeast extract, nutrient broth, dextrose (cerelose), white potato dextrin, soy flour, peptone and other components known in the art. Typical buffers are phosphate buffer (e.g., 0.1 M at pH 7), MES (2-[N-morpholino]ethanesulfonic acid), Bis-Tris (bis[2-hydroxyethyl]iminotris[hydroxymethyl]-methane), PIPES (1,4-piperazine-diethanesulfonic acid), HEPES (N-[2-hydroxyethyl]piperazine-N'-[2-ethanesulfonic acid]), TRIS (tris(hydroxymethyl)aminomethane) and MOPS (3-[N-morpholino]propanesulfonic acid) buffer (e.g., 0.1 M at pH 7). Typical solvents are acetonitrile, acetone, ethyl ether, isopropanol, t-butanol, isoamyl alcohol, p-dioxane, isopropyl ether, dimethyl sulfoxide, t-butyl methyl ether (TBME), toluene, tetrahydrofuran and CH₂Cl₂. Preferably, the microbial reduction is carried out in fermentation media.

The duration of the chiral reduction reaction may vary from about 18 to about 96 hours, and is preferably about 48-72 hours.

At the end of the reduction reaction, the hydroxy compound of formula I may be extracted by well known methods, using organic solvents such as ethyl acetate (EtOAc), t-butyl methyl ether (TBME), methylene chloride (CH₂Cl₂) and the like. Adsorption to resins, chromatography, and other physical methods known to the art may also be used to extract the hydroxy compound of formula I.

A large number of microorganisms were investigated to determine whether or not they reduce the ketone compound of formula II. Many such microorganisms failed to provide the desired specificity or productivity.

The examples below demonstrate the evaluation of microorganisms in the reduction of this invention and the preparation of milligram quantities of the hydroxy compound of formula I.

### Example 1

The general method for identifying the stereoselective microbial reduction of the compound of formula II for use as a synthetic precursor for the production of the compound of formula I is described below.

Seed cultures of yeast, filamentous fungi, and bacteria were grown in 125 ml or 300 ml flasks containing 25 ml or 50 ml of YPD (1% yeast extract, 2% peptone, 2% dextrose; pH 5.5), SIM6 (3.5% soy flour, 5% white potato dextrin, 0.5% cerelose, 2 mg/l cobalt chloride, 0.5% calcium carbonate; pH 6.0) and NYC (0.8% nutrient broth, 2% yeast extract, 1.1% cerelose; pH 7.0) media, respectively, for 72 hours at 30°C with agitation (175-250 rpm) prior to inoculation (4 % v/v) into flask fermentations (25ml YPD/125 ml flask for yeast and filamentous fungi or 25ml NYC /125 ml flask for bacteria) which were incubated at 30°C with agitation (250 rpm). In all fermentations, medium pH was adjusted prior to inoculation but was not controlled during culture propagation and ketone reduction. Reduction was initiated by adding 0.5-1.0 g/I of the ketone compound of formula II dissolved in ethanol (25 mg/ml) directly to cultures following 24 hours of growth. Samples of fermentation broth extracted with EtOAc (1:1) following 48 hours incubation with substrate were analyzed by reverse-phase HPLC. Cultures demonstrating consistent reduction activity without significant substrate degradation following repeated fermentations using this procedure were further analyzed by chiral HPLC to determine the configuration of the product alcohol. Cultures capable of reducing the ketone of formula II at 1.0 g/l in high enantiomeric excess yielding the hydroxy compound of formula I (the S enantiomer), are summarized in Table 1.

**Table 1. Microorganisms capable of selectively reducing Compound II to Compound I at 1.0 all.**

| **Culture** | **Strain #** | **% EE, *S*/*R*** | **% Yield** |
|---|---|---|---|
| Aspergillus niveus | 12276 | 100 *S* | 7 |
| Curvularia lunata | 34477 | 100 *S* | 18 |
| Mucor racemosus | 7924 | 100 *S* | 4 |
| Mucor circinelloides | 1207a | 100 *S* | 9 |
| Saccharomyces cerevisiae | Y-2034 | 100 *S* | 8 |
| Saccharomyces uvarum | 10613 | 100 *S* | 11 |
| | 32634 | 100 *S* | 7 |
| Pichia methanolitica | 58403 | 84 *S* | 24 |
| Torulaspora fermentati | 20100 | 100 *S* | 5 |
| Torulaspora species | 66815 | 100 *S* | 14 |
| Neurospora crassa | 14692 | 76 *S* | 4 |
| Rhodococcus erythropolis | 25544 | 100 *S* | 6 |
| Rhodococcus fascians | 202210 | 100 *S* | 46 |
| Rhodococcus rhodochrous | 999 | 100 *S* | 12 |
| | 21243 | 100 *S* | 12 |
| | 29670 | 100 *S* | 13 |
| | 29675 | 100 *S* | 8 |
| Rhodococcus species | 19071 | 100 *S* | 10 |
| | 19148 | 100 *S* | 8 |
| Geotrichum candidum | 74487 | 100 *S* | 25 |
| Doratomyces stemonitis | SPR 423 | 100 *S* | 11 |
| Scytalidium lignacola | SPR 531 | 89 *S* | 30 |
| Mortierella isabellina | SPR 875 | 57 *S* | 35 |

### Example 2

The general method for investigating the fermentation parameters for the reduction of the ketone compound of formula II by *Rhodococcus fascians* ATCC No. 202210 or fungal isolate *Geotrichum candidum* ATCC No. 74487 capable of reducing compound II at concentrations greater than those used in Example 1 is described below.

Seed culture propagation and bioconversions employing *Rhodococcus fascians* ATCC No. 202210 and *Geotrichum candidum* ATCC No. 74487 were conducted in 125 ml flasks containing 25 ml of NYC, YPD, SIM6 or TGP (1% Tastone 154, 2% glycerol, 1% potassium phosphate dibasic, pH 7.0) media for 24-72 hours at 30°C with agitation (250 rpm) prior to inoculation (4 % v/v) into 125 ml flasks containing 25 ml of bioconversion media as summarized in Tables 2 and 3. In all fermentations, medium pH was adjusted prior to inoculation but was not controlled during culture propagation and ketone reduction. Reduction was initiated by adding ketone compound of formula II at 1-10 g/l dissolved in ethanol or dimethyl sulfoxide (DMSO) (25-50 mg/ml) directly to cultures following 24-48 hours of growth. In bioconversions using cell concentrates, cultures were isolated by centrifugation (8000 rpm X 10 min.) following 24-48 hours of growth and resuspended in fresh media as indicated prior to the addition of ketone. Samples of fermentation broth extracted with EtOAc (1:1) or TBME (1:1) following 48-96 hours incubation with substrate were analyzed by reverse-phase HPLC to assess yield; analysis by chiral HPLC was conducted to confirm selective synthesis of the S enantiomer product (compound of formula I) in high enantiomeric excess.

**Table 2. Effect of bioconversion parameters on productivity of R. fascians ATTC No. 20221b.**

| **Seed Propagation conditions: 30°C, 250 rpm** | **Bioconversion Conditions (25 ml media/125 ml flask, 250 rpm)** | **% Yield** |
|---|---|---|
| 25 ml NYC /125 ml flask | 1 g/l: YPD, 30°C | 41 |
| 24 hours (4% v/v transfer) | 2 g/l: YPD, 30°C | 32 |
| 25 ml YPD /125 ml flask | 1 g/l: YPD, 30°C | 50 |
| 24 hours (4% v/v transfer) | 2 g/l: YPD, 30°C | 42 |
| | 1 g/l: NYC, 25°C | 45 |
| 25 ml NYC /125 ml flask | 1 g/l: NYC, 30°C | 42 |
| 7.2 hours (4% v/v transfer) | 1 g/l: YPD, 30°C | 47 |
| | 1 g/l: NYC, 35°C | 48 |
| | 2 g/l: NYC, 25°C | 44 |
| | 2 g/l: NYC, 30°C | 43 |
| | 2 g/: YPD, 30°C | 44 |
| | 2 g/l: NYC, 35°C | 39 |
| 25 ml TGP / 125 ml flask | 1 g/1: TGP, 30°C | 69 |
| 24 hours (4% v/v transfer) | 2 g/1: TGP, 30°C | 64 |
| | 4 g/l: TGP, 30°C | 28 |
| | 10 g/l: TGP, 30°C | 11 |
| 25 ml TGP /125 ml flask | 4 g/l: 5X cell concentrate, TGP, 30°C | 68 |
| 24 hours (4% v/v transfers | 10 g/l: 5X cell concentrate, TGP, 30°C | 31 |

| | | |
|---|---|---|
| Ketone compound of formula II dissolved in ethanol (25-50 mg/ml) added at 1-10 g/l where indicated following 24 hours of growth. | | |

**Table 3. Effect of bioconversion parameters on productivity of G. candidum ATCC No. 74487.**

| **Seed Propagation conditions** | **Bioconversion Conditions (25 ml media/125 ml flask)** | **% Yield** |
|---|---|---|
| 25 ml SIM-6 /125 ml flask, 30°C, 250 rpm 72 hours (4% v/v transfer) | 2 g/l: TGP, 30°C | 18 |
| | 4 g/l: TGP, 30°C | 9 |
| | 10 g/l: TGP, 30°C | 6 |
| | | |
| | 2 g/l: YPD, 30°C | 33 |
| | 2 g/l: YPD, 35°C | 39 |
| | | |
| | 2 g/l: TNC, 30°C | 38 |
| | 2 g/l: TNC, 35°C | 45 |
| | | |
| | 2 g/l: TN2C, 30°C | 54 |
| | 2 g/l: TN2C, 35°C | 46 |

| | | |
|---|---|---|
| Ketone compound of formula II dissolved in DMSO (25-50 mg/ml) added at 2-10 g/l following 24-48 hours of growth. TNC medium: 1% Tastone 154, 2% NZ-amine, 3% cerelose, pH 5.5. TN2C medium: TNC medium with 6% cerelose. | | |

### Example 3

Milligram quantities of the hydroxy compound of formula I derived from the stereoselective reduction of ketone compound of formula II were prepared using *Rhodococcus fascians* ATCC No. 202210 and fungal isolate *Geotrichum candidum* ATCC No. 74487 in multiple flask fermentations employing conditions summarized in Tables 2 and 3. Following 72-96 hours of incubation, fermentation broths of each of the cultures were pooled prior to centrifugation to isolate the cells which harbor most of the product and residual substrate. The cell pellets were extracted with TBME (10-20 volumes/wet weight). Anhydrous MgSO₄ was added to the TBME extract to remove residual water, the extract was filtered and the filtrate concentrated by evaporation.

Extract concentrate was subjected to purification by preparative thin layer chromatography employing 10-20 GF silica plates (20cm X 20cm X 1000 micron) and developed with a solution of EtOAc:hexane (50:50). Material comigrating with the desired product was scraped from each of the silica plates, pooled and eluted from the silica with TBME which was subsequently evaporated to dryness. Approximately 170 mg of product derived from 450-600 mg of ketone compound of formula II was isolated from each culture bioconversion. Isolated material was confirmed to be the desired hydroxy compound of formula I by reverse phase and chiral HPLC, NMR, and mass spectrum analyses.

## Claims

1. A process for the stereoselective reduction of 1-(4-fluorophenyl)-3(R)-[3-oxo-3-(4-fluorophenyl)propyl]-4(S)-(4-hydroxyphenyl)-2-azetidinone to 1-(4-fluorophenyl)-3(R)-[3(5)-hydroxy-3-(4-fluorophenyl)-propyl]-4(S)-(4-hydroxyphenyl)-2-azetidinone comprising adding 1-(4-fluoro-phenyl)-3(R)-[3-oxo-3-(4-fluorophenyl)-propyl]-4(S)-(4-hydroxyphenyl)-2-azetidinone to a microorganism in medium, medium and buffer, medium and solvent, or medium and a mixture of buffer and solvent, incubating the resulting mixture, and isolating 1-(4-fluoro-phenyl)-3(R)-[3(S)-hydroxy, 3-(4-fluorophenyl)-propyl]-4(S)-(4-hydroxyphenyl)-2-azetidinone,
wherein the microorganism is of a species selected from the group consisting of Aspergillus niveus, Curvularia lunata, Mucor racemosus and circinelloides, Saccharomyces cerevisiae and uvarum, Pichia methanolica, *Torulaspora fermentati* and *species*, *Neurospora crassa* and *Rhodococcus erythropolis, fascians, rhodochrous* and *species*, Geotrichum candidum, Doratomyces stemonitis, Scytalidium lignicola and Mortierella isabellina.

2. A process of claim 1 wherein the microorganism is *Rhodococcus fascians* or fungal isolate *Geotrichum candidum.*

3. A process of claim 1 wherein the microorganism is *Rhodococcus fascians.*

## Patentansprüche

1. Ein Verfahren zur stereoselektiven Reduktion von 1-(4-Fluorphenyl)-3(R)-[3-oxo-3-(4-fluorphenyl)propyl]-4(S)-(4-hydroxyphenyl)-2-azetidinon zu 1-(4-Fluorphenyl)-3(R)-[3(S)-hydroxy-3-(4-fluorphenyl)propyl]-4(S)-(4-hydroxyphenyl)-2-azetidinon, umfassend die Zugabe von 1-(4-Fluorphenyl)-3(R)-[3-oxo-3-(4-fluorphenyl)propyl]-4(S)-(4-hydroxyphenyl)-2-azetidinon zu einem Mikroorganismus in Medium, Medium und Puffer, Medium und Lösungsmittel oder Medium und einer Mischung aus Puffer und Lösungsmittel, das Inkubieren der resultierenden Mischung und das Isolieren von 1-(4-Fluorphenyl)-3(R)-[3(S)-hydroxy-3-(4-fluorphenyl)propyl]-4(S)-(4-hydroxyphenyl)-2-azetidinon,
wobei der Mikroorganismus eine Spezies ist, ausgewählt aus der Gruppe, bestehend aus Aspergillus niveus, Curvularia lunata, Mucor racemosus und circirielloides, Saccharomyces cerevisiae und uvarum, Pichia methanolica, *Torulaspora fermentatz* und *spec., Neurospora crassa* und *Rhodococcus erythropolis, fascians, rhodochrous* und *spec.,* Geotrichum candidum, Doratomyces stemonitis, Scytalidium lignicola und Mortierella isabellina.

2. Ein Verfahren nach Anspruch 1, wobei der Mikroorganismus *Rhodococcus fascians* oder Pilzisolat *Geotrichum candidum* ist.

3. Ein Verfahren nach Anspruch 1, wobei der Mikroorganismus *Rhodococcus fascians* ist.

## Revendications

1. Procédé de réduction stéréosélective de 1-(4-fluorophényl)-3-(R)-[3-oxo-3-(4-fluorophényl)propyl]-4(S)-(4-hydroxyphényl)-2-azétidinone en 1-(4-fluorophényl)-3-(R)-[3(S)-hydroxy-3-(4-fluorophényl)-propyl]-4(S)-(4-hydroxyphényl)-2-azétidinone, comprenant ajouter de la 1-(4-fluorophényl)-3(R)-[3-oxo-3-(4-fluorophényl)-propyl]-4(S)-(4-hydroxyphényl)-2-azétidinone à un micro-organisme dans un milieu, milieu et tampon, milieu et solvant ou milieu et un mélange de tampon et de solvant, incuber le mélange résultant et isoler la 1-(4-fluorophényl)-3(R)-[3(S)-hydroxy-3-(4-fluorophényl)-propyl]-4(S)-(4-hydroxyphényl)-2-azétidinone,
où le micro-organisme est d'une espèce sélectionnée parmi le groupe consistant en *Aspergillus niveus, Curvularia lunata, Mucor racemosus* et *circinelloides, Saccharomyces cerevisiae* et *uvarum, Pichia methanolica, Torulaspora fermentati* et *species, Neurospora crassa* et *Rhodococcus erythropolis, fascians, rhodochrous* et *species, Geotrichum candidum, Doratomyces stemonitis, Scytalidium lignicola* et *Mortierella isabellina.*

2. Procédé selon la revendication 1, dans lequel le micro-organisme est *Rhodococcus fascians* ou un isolat fongique *Geotrichum candidum.*

3. Procédé selon la revendication 1, dans lequel le micro-organisme est *Rhodococcus fascians.*
